# EUROPEAN PATENT APPLICATION

(11) **EP 4 841 510 A1**
(43) Date of publication of application: **18.11.2026**
(21) Application number: 25176687.9
(22) Date of filing: 15.05.2025
(51) Int. Cl.: G06N 3/0475

(54) **METHOD FOR GENERATING TABULAR MEDICAL DATA**

(71) Applicant: Botdesign, 31400 Toulouse (FR)
(72) Inventor: Alassonniere, Stéphanie, 92340 BOURG LA REINE (FR); Chadebec, Clément, 92340 BOURG LA REINE (FR); Thuillart, Olivier, 31400 TOULOUSE (FR); Fontaine, Raphaël, 31300 TOULOUSE (FR); Fraysse, Jean Louis, 31400 TOULOUSE (FR)
(74) Representative: Plasseraud IP

(57) **Abstract**

The invention relates to a method for generating tabular medical data using a Riemannian Hamiltonian Variational Auto Encoder (RHVAE), said method comprising a training phase (TP),
said RHVAE comprising said RHVAE comprising an encoder configured to project said tabular medical data into a latent space, and a decoder configured to reconstruct tabular medical data from latent representations,
said tabular data comprising tabular variables, continuous variables and/or categorical variables,
the training phase comprising:
- defining a composite loss function adapted to the different types of tabular variables, said composite loss comprising a first loss function, for example an L1 loss, for continuous variables, a second loss function, for example a cross-entropy loss, for categorical variables, a third loss function, for example a binary cross-entropy loss for binary variables,
- training said RHVAE by jointly
-- optimizing (S2) weights of said encoder and decoder using said composite loss function, and
-- learning (S3) a Riemannian metric on the latent space so as to capture the intrinsic geometry of said tabular medical data.

## Description

### Technical Field

The present document relates to the field of artificial intelligence and machine learning, specifically to data augmentation techniques utilizing generative models. More precisely, the invention concerns methods employing variational autoencoders (VAEs) combined with Riemannian geometry for generating synthetic tabular medical data. This technology is particularly applicable to clinical data, including but not limited to continuous, categorical, and binary patient variables, and is especially useful in scenarios involving small, incomplete, or sensitive datasets commonly encountered in medical studies, diagnostics, and clinical trials. Exemplary use cases for the generated synthetic data include, but are not limited to, fundamental and clinical research, training artificial intelligence models (e.g., for prediction or classification), and aiding in the creation or validation of new digital medical devices.

### Prior art

Variational Autoencoders (VAEs) are generative artificial intelligence models widely employed for creating synthetic data in various applications, notably image generation. A conventional VAE typically includes an encoder neural network that compresses high-dimensional input data into a lower-dimensional latent space, and a decoder neural network that reconstructs the data from this latent representation. Standard VAEs optimize the weights of these networks by minimizing a reconstruction loss combined with a Kullback-Leibler divergence term to regularize the latent space.

However, standard VAEs assume an isotropic Gaussian latent space that does not necessarily reflect the true geometry or distribution of complex data. In particular, for tabular medical datasets, which usually consist of heterogeneous data types-such as continuous (e.g., blood pressure, biomarker concentrations), categorical (e.g., blood type, disease stage), and binary variables (e.g., smoker/non-smoker)-standard VAEs fail to adequately capture and reproduce the intrinsic statistical distributions and correlations among these variables.

Existing techniques like Synthetic Minority Over-sampling Technique (SMOTE) have been employed to handle imbalanced tabular datasets. While useful, SMOTE and related interpolation methods oversimplify data generation by linearly interpolating minority class samples, thereby often distorting statistical characteristics. Similarly, Generative Adversarial Networks (GANs), although powerful for generating realistic images, typically suffer from training instability and mode-collapse when applied to heterogeneous tabular data.

Recent approaches have introduced geometry-based VAEs, notably the Riemannian Hamiltonian Variational Autoencoder (RHVAE), which incorporate a learned Riemannian metric into the latent space. Such approaches allow a more faithful representation of complex data structures, enabling more meaningful sampling and data generation, especially for small-sized and high-dimensional datasets. However, previously published RHVAE methods primarily address image data and do not provide solutions specifically tailored for tabular medical data with mixed variable types and missing values.

There is thus a need for an improved VAE-based data generation method specifically adapted for medical tabular data that simultaneously considers different variable types, manages missing values effectively, and utilizes learned geometric structures within the latent space to better reflect the intrinsic distribution of real medical datasets.

### Summary

To this end, the present document concerns a computer-implemented method for generating tabular medical data using a Riemannian Hamiltonian Variational Auto Encoder (RHVAE), said method comprising a training phase,
said RHVAE comprising said RHVAE comprising an encoder configured to project said tabular medical data into a latent space, and a decoder configured to reconstruct tabular medical data from latent representations,
said tabular data comprising tabular variables, continuous variables and/or categorical variables,
the training phase comprising:
   - defining a composite loss function adapted to the different types of tabular variables, said composite loss comprising a first loss function, for example an L1 loss, for continuous variables, a second loss function, for example a cross-entropy loss, for categorical variables, a third loss function, for example a binary cross-entropy loss for binary variables,
   - training said RHVAE by jointly
      -- optimizing weights of said encoder and decoder using said composite loss function, and
      -- learning a Riemannian metric on the latent space so as to capture the intrinsic geometry of said tabular medical data.

A Riemannian Hamiltonian Variational Autoencoder (RHVAE) is a generative artificial intelligence model combining the principles of a Variational Autoencoder (VAE) with Riemannian geometry and Hamiltonian Monte Carlo (HMC) sampling. Like a standard VAE, the RHVAE comprises two main neural networks: an encoder, which compresses input data into a lower-dimensional latent space, and a decoder, which reconstructs the original data from the latent representations. However, the RHVAE differs significantly in its modeling of the latent space. Rather than assuming a simple Euclidean latent structure, the RHVAE treats the latent space as a Riemannian manifold with a learned metric. This metric is specifically trained to capture the complex geometry and statistical distributions inherent in the original data, allowing the latent space to adaptively reflect intrinsic data patterns.

The RHVAE leverages Hamiltonian Monte Carlo (HMC) sampling within this learned Riemannian geometry to explore the latent space efficiently. By incorporating information about the local curvature and density captured by the learned metric, the HMC sampling procedure guides the latent-space exploration toward regions of high data likelihood, enabling the generation of more realistic and statistically coherent synthetic data. In practical terms, this approach addresses limitations of classical VAEs-such as overly simplistic assumptions about latent-space structure-by providing a richer, geometry-aware representation. Thus, the RHVAE is particularly suited for challenging applications such as generating synthetic tabular medical data, where faithfully reproducing complex distributions and mixed data types (continuous, categorical, binary) is critical for downstream predictive modeling and clinical applications.

Such RHVAE is for example known from Chadebec, C., Thibeau-Sutre, E., Burgos, N., & Allassonnière, S. (2022). Data Augmentation in High Dimensional Low Sample Size Setting Using a Geometry-Based Variational Autoencoder. IEEE Transactions on Pattern Analysis and Machine Intelligence. DOI: 10.1109/TPAMI.2022.3185773.

The training phase corresponds to the step of adjusting and optimizing the internal parameters of the RHVAE based on provided input tabular medical data. During this phase, input tabular medical data are fed into the RHVAE. The RHVAE parameters, including those defining the encoder, the decoder, and the structure of the latent space, are iteratively adjusted to minimize a predefined loss function. The result of this phase is a trained model capable of accurately capturing statistical patterns present in the original medical data.

Tabular medical data refers to structured healthcare datasets organized into rows representing individual patients, clinical records, or distinct medical observations, and columns corresponding to multiple patient attributes. Such data may originate from various sources, including but not limited to biological data (e.g., lab results, genetics), data collected during care provision (e.g., diagnoses, treatments, vital signs collected within a care context), prevention data, and data from digital medical devices. Each attribute, often called a variable, encodes specific types of medical, demographic, biological, genetic, or diagnostic information. Due to the diversity and complexity of medical information, these datasets frequently include heterogeneous data types such as continuous, categorical (nominal or ordinal), binary variables, and may also exhibit missing or incomplete data entries.

Continuous variables are numerical measures that exist on a continuous spectrum, typically representing biological measurements, physiological parameters, or clinical scores. They can assume any numeric value within a given interval and often follow particular statistical distributions (normal, skewed, etc.). Examples include:
- Demographic and Anthropometric Measurements: Patient age (in years), body weight (in kilograms), height (in centimeters), Body Mass Index (BMI, calculated from weight and height).
- Vital Signs and Physiological Measures: Systolic and diastolic blood pressure (mmHg), heart rate (beats per minute), respiratory rate (breaths per minute), body temperature (degrees Celsius), oxygen saturation (percentage SpO₂).
- Biochemical and Laboratory Test Results: Blood glucose concentration (mg/dL or mmol/L), cholesterol levels including high-density lipoprotein (HDL) and low-density lipoprotein (LDL), electrolytes such as sodium (Na⁺), potassium (K⁺), calcium (Ca²⁺), liver function enzymes (ALT, AST), kidney function parameters (creatinine, blood urea nitrogen).
- Clinical Scores and Indices: Severity indices such as APACHE II or SOFA scores for intensive care patients, cardiovascular risk scores (Framingham score), pain scales (visual analog scale or numeric rating scale from 0 to 10), inflammation markers (e.g., CRP levels).

Categorical variables represent qualitative information by grouping data points into discrete categories. These variables can be nominal (without inherent numerical order) or ordinal (with clearly defined ordering or hierarchy).

Nominal categorical variables do not have a natural order among their categories and represent qualitative distinctions between patient attributes, diseases, or treatments. Examples include:
- Demographic Characteristics: Gender (male, female, non-binary), ethnicity or racial classification (e.g., Caucasian, African-American, Asian, Hispanic), marital status (single, married, divorced, widowed).
- Clinical and Diagnostic Information: Blood type (A, B, AB, O), diagnostic categories according to ICD-10 codes (e.g., type of diabetes, type of infection), treatment modalities (type of chemotherapy, radiotherapy, surgical procedure), pathogens or microorganisms identified (strain of influenza virus, bacterial species).
- Genetic and Molecular Information: Genotype groups (e.g., heterozygous/homozygous status for certain genetic variants), presence or absence of genetic mutations or markers (e.g., BRCA1/BRCA2 mutation).

Ordinal categorical variables contain distinct categories with a clear ranking or hierarchy among categories. They often encode severity, progression stages, or levels of clinical symptoms. Examples include:
- Disease Severity and Clinical Staging: Cancer stages (Stage I, Stage II, Stage III, Stage IV), severity classifications (mild, moderate, severe, critical), level of cognitive impairment (normal cognition, mild cognitive impairment, moderate dementia, severe dementia).
- Symptom Intensity or Pain Scales: Pain rating scales (e.g., none, mild, moderate, severe, very severe), Likert-scale ratings for patient-reported outcomes (1 = strongly disagree to 5 = strongly agree), frequency of symptoms (never, rarely, sometimes, often, always).
- Functional and Quality-of-life Measures: Mobility status (fully independent, partially dependent, fully dependent), performance status scales (e.g., ECOG performance status: grade 0 to 4, from fully active to completely disabled).

Binary variables represent attributes or clinical outcomes that have exactly two possible states, typically coded as 0/1, yes/no, true/false, or presence/absence. Binary variables simplify categorical distinctions into two clear groups. Examples in medical datasets include:
- Risk Factors and Lifestyle: Smoking status (smoker/non-smoker), alcohol consumption (regular drinker/non-drinker), physical activity (sedentary/active).
- Clinical Diagnoses and Outcomes: Presence or absence of a disease (e.g., diabetic/non-diabetic), positive or negative test results (e.g., COVID-19 PCR test positive or negative, presence or absence of specific antibodies).
- Treatment and Intervention Indicators: Whether a patient received a specific treatment or not (received/not received chemotherapy), surgery performed or not performed (yes/no), clinical response or remission achieved (responder/non-responder).
- Patient Compliance and Follow-up: Medication adherence (adherent/non-adherent), attendance to follow-up appointments (attended/missed).

To enable the calculation of the composite loss function defined, the decoder network within the RHVAE may be specifically configured and adapted during the training phase to output data corresponding to the different variable types. For continuous variables, the decoder may output raw numerical values suitable for comparison using the first loss function (e.g., L1 loss). For categorical variables, the decoder may output logits representing the probability distribution across categories, suitable for the second loss function (e.g., cross-entropy loss). For binary variables, the decoder may typically output logits which are then passed through an activation function, such as a sigmoid function, to produce probabilities between 0 and 1, suitable for comparison using the third loss function (e.g., binary cross-entropy loss). This adaptation ensures that the reconstructed data generated by the decoder aligns structurally with the heterogeneous input data and allows for the effective minimization of the composite loss function.

Medical datasets frequently contain incomplete records or missing values. Data may be missing due to patient dropout, measurement error, privacy constraints, or logistical challenges in data collection. Missing data may be explicitly marked using masks or indicators to distinguish between observed and unobserved values.

Medical tabular data may originate from various medical data acquisition sensors.

Said method may comprise a step of acquiring initial raw medical data using said sensors before it is formatted into a tabular structure.

As used herein, the term sensors broadly refers to technical devices or components designed to detect specific physical, chemical, or biological quantities or changes in a patient's body or environment. These sensors convert measured parameters, such as pressure, temperature, electrical potential, light intensity, chemical concentration, or motion, into signals (often electrical) that can be further processed, quantified, and recorded as data, forming the basis of technical measurements.

Said sensors can be generally categorized based on the type of system they are part of. Such categories include, for example: physiological monitoring systems (prominently featuring sensors), medical imaging systems (utilizing specialized detectors, a form of sensor), laboratory analysis systems (employing various physical/chemical detection techniques), genetic and molecular analysis systems, and clinical classification and record systems (aggregating data often derived from the previous categories).

Physiological Monitoring Systems represent a primary category utilizing sensors often directly applied to or near the patient for measuring physiological and vital signs. Examples of such sensors and systems include: electrocardiogram (ECG) devices sensing electrical heart activity via electrodes; pulse oximeters sensing blood oxygen saturation using light emitters and detectors; heart-rate monitors detecting cardiac pulse; blood pressure monitors sensing arterial pressure (non-invasively via cuffs or invasively via transducers); respiratory rate sensors detecting breathing movements or airflow; thermometers measuring body temperature; and other continuous physiological monitoring systems like continuous glucose monitors (electrochemical sensors) or electroencephalogram (EEG) devices (electrodes). The signals captured by these sensors are technically processed into quantifiable numerical values (e.g., heart rate, SpO₂, blood pressure readings, temperature values) or derived features (e.g., specific ECG intervals), constituting continuous or categorical data entries.

Medical Imaging Systems technically capture anatomical or functional information. Examples include: Computed Tomography (CT) devices using X-ray detectors; Magnetic Resonance Imaging (MRI) devices detecting radiofrequency signals from nuclear spins; Positron Emission Tomography (PET) scanners detecting gamma rays from positron annihilation; X-ray radiography systems using film or digital detectors; ultrasound scanners emitting and detecting sound wave echoes; or other imaging modalities. The detectors within these systems act as sensors capturing physical phenomena. From the resulting technically acquired images, quantitative measurements are derived, such as tumor volumes, lesion sizes, intensity metrics (e.g., Hounsfield units, signal intensity values), radiomic features, or tissue property estimations, which are then structured into tabular format.

Laboratory Analysis Systems comprise laboratory analyzers and diagnostic instruments performing technical assays on biological samples. Examples include: instruments performing biochemical assays (e.g., spectrophotometers, chemical analyzers); hematology analyzers (e.g., cell counters using electrical impedance or flow cytometry); immunoassay platforms (e.g., ELISA readers using optical detection); genetic sequencing platforms; polymerase chain reaction (PCR) machines (often using fluorescence detection); or other biochemical measurement devices. These instruments provide continuous laboratory variables such as blood glucose, cholesterol levels, electrolytes, liver enzymes, or kidney function biomarkers, or categorical/binary results based on detected thresholds or presence/absence signals, all derived from technical detection processes within the instruments.

Genetic and Molecular Analysis Systems include genomic and molecular biology measurement systems used to technically analyze biological material. Examples include: DNA/RNA sequencing devices reading nucleotide sequences; microarrays measuring gene expression optically; proteomic analyzers (e.g., mass spectrometers) identifying/quantifying proteins; and similar instruments identifying the presence or absence or quantity of specific genetic or molecular markers. These technical analyses yield data such as specific gene variants (categorical/binary), expression levels (continuous), or protein quantities (continuous), forming technically derived tabular data.

A loss function, also referred to as an objective function or cost function, is a mathematical formulation used in machine learning and statistical modeling to measure how well a predictive model performs by quantifying the discrepancy between predicted outputs and actual, observed data. The loss function thus serves as a criterion that the learning algorithm seeks to minimize during training. Specifically, it assigns a numerical value-referred to as a "loss"-to a given set of predictions, reflecting their deviation from the true observations.

The first loss for continuous variables may be an L1 loss (mean absolute error), an L2 loss (mean squared error), or a Huber loss.

The second loss for categorical variables may be a cross-entropy loss or a negative log-likelihood loss.

The third loss for binary variables may be a binary cross-entropy loss, a logistic loss, or a hinge loss.

Optimizing weights refers to the process of adjusting numerical parameters within a neural network (in this case, the RHVAE's encoder and decoder networks) to minimize the predefined composite loss function. This adjustment is typically done using iterative optimization algorithms, such as stochastic gradient descent (SGD) or its variants (Adam, RMSProp, etc.). The purpose of optimization is to iteratively update the network parameters ("weights") until the model's predictions closely match the observed training data, achieving minimal discrepancy as measured by the loss function.

Jointly optimizing indicates simultaneously updating multiple sets of parameters-in this context, both the neural network weights (of encoder and decoder) and the parameters defining the Riemannian metric. Joint optimization ensures that these parameters are learned simultaneously, interacting with each other during training, and leading to a coherent representation and reconstruction of data. This contrasts with sequential or independent training, where components are optimized separately in stages.

A Riemannian metric is a mathematical tool that assigns a notion of distance, angles, and volumes to a smooth manifold-in this context, the latent space of the RHVAE. Unlike a simple Euclidean metric that assumes a flat geometry, a Riemannian metric can capture curvature and local geometry variations within the latent space. By learning this metric from data, the latent space better reflects the intrinsic geometrical structure and statistical distribution of the medical data, enabling more accurate modeling and sampling.

Intrinsic geometry pertains to the inherent geometric and statistical structure that underlies the data distribution. Rather than treating data points simply as vectors in a Euclidean space, intrinsic geometry considers the relationships and distances between data points based on a manifold's curvature and local topology. Capturing the intrinsic geometry means modeling how data naturally cluster, align, or distribute, improving the model's capacity to represent and generate realistic synthetic data consistent with the underlying medical reality.

By employing a composite loss function tailored to continuous, categorical, and binary variables, the generated datasets more accurately reflect the true statistical properties of real clinical data. Furthermore, jointly training the encoder, decoder, and a Riemannian metric ensures that the latent space effectively captures the intrinsic geometric structure of medical data, enabling more realistic and clinically coherent synthetic data generation.

The composite loss function may be a weighted sum of the first, second and third loss.

Said Riemannian metric may guide a Hamiltonian Monte Carlo (HMC) sampling procedure in the latent space, so that the sampling path favors high-density regions corresponding to the distribution of the tabular medical data.

A sampling procedure is a process used to select or generate representative points from a statistical distribution. In the context of latent spaces, the sampling procedure generates latent representations that, when decoded, yield new synthetic data points.

Hamiltonian Monte Carlo (HMC) is an advanced sampling method used to efficiently explore high-dimensional probability distributions. By simulating Hamiltonian dynamics-commonly found in physics and involving positions and momenta-HMC can effectively move through the latent space. Utilizing gradient information from the learned distribution, HMC ensures more informed and efficient sampling compared to simple random walks, significantly improving sample quality.

The sampling path refers to the trajectory or route taken by the sampling algorithm (here, HMC) through the latent space during the sampling procedure. The path guides how latent points are selected and ensures that sampled latent points are concentrated in regions of higher probability density, reflecting realistic and statistically meaningful representations of the data.

High-density regions are areas within a probability distribution where data points occur most frequently or have the highest likelihood. In the context of medical tabular data generation, these regions represent latent-space clusters corresponding closely to realistic patient profiles and typical clinical scenarios. Sampling from these regions increases the fidelity and clinical relevance of the generated synthetic data.

The distribution of tabular medical data refers to the underlying statistical patterns, correlations, and relationships among variables in real-world medical datasets.

Such sampling procedure is equivalent to the sampling described in Chadebec, C., Thibeau-Sutre, E., Burgos, N., & Allassonnière, S. (2022). Data Augmentation in High Dimensional Low Sample Size Setting Using a Geometry-Based Variational Autoencoder. IEEE Transactions on Pattern Analysis and Machine Intelligence. DOI: 10.1109/TPAMI.2022.3185773.

The tabular medical data may be identified via a mask and excluded from the reconstruction error calculation in said composite loss function.

Optimizing the encoder and decoder may comprise applying a KL-divergence regularization term between a learned latent distribution and a predefined prior, the Riemannian metric being updated in parallel to reflect the curvature of the latent space based on said tabular medical data.

The Kullback-Leibler (KL) divergence regularization term is a mathematical measure used to quantify how closely the latent distribution learned by the encoder (the posterior distribution) matches a predefined distribution known as the prior distribution. In the context of VAEs, this prior distribution is typically chosen to be a simple standard Gaussian (normal distribution). Minimizing the KL divergence ensures the latent space maintains a structured and regular geometry, facilitating meaningful generation and sampling from the latent representations.

A latent distribution refers to the probability distribution of the encoded representations of the input data within the latent space. This distribution is learned during training, meaning its parameters (such as mean and variance) are adjusted to optimally represent the observed data.

A predefined prior is a fixed, chosen probability distribution imposed on the latent space to regularize and structure the latent representation learned by the encoder. Typically, the prior is a simple standard Gaussian distribution with zero mean and unit variance, which provides a stable reference point and ensures regularity and coherence within the latent representation, simplifying subsequent sampling and generation processes.

The composite loss function may be defined by the following equation: $\begin{matrix}{L}_{composite} = α ⋅ {M}_{cont} ⋅ {L}_{cont} \left({x}_{cont}, \hat{{x}_{cont}}\right) + β ⋅ {M}_{cat} ⋅ {L}_{cat} \left({x}_{cat}, \hat{{x}_{cat}}\right) + γ ⋅ {M}_{bin} \\ ⋅ {L}_{bin} \left({x}_{bin}, \hat{{x}_{bin}}\right) + δ ⋅ {D}_{KL} \left({q}_{ϕ}\left(z\left|x\right.\right)\left‖p\left(z\right)\right.\right)\end{matrix}$ where:
- the first term $α ⋅ {M}_{cont} ⋅ {L}_{cont} \left({x}_{cont}, \hat{{x}_{cont}}\right)$ is a loss term specifically adapted for continuous variables. It measures discrepancies between observed continuous data values x_{cont} and reconstructed continuous values $\hat{{x}_{cont}}$. The binary mask M_{cont} ensures this term is calculated only from observed values, and α is a weighting coefficient that adjusts the relative importance of this continuous loss term.
- the second term $β ⋅ {M}_{cat} ⋅ {L}_{cat} \left({x}_{cat}, \hat{{x}_{cat}}\right)$ is a loss term specifically adapted for categorical variables. It quantifies differences between observed categorical data x_{cat} and predicted categorical outputs $\hat{{x}_{cat}}$. The mask M_{cat} restricts calculations to observed values, and the coefficient β adjusts the influence of categorical variables within the composite loss.
- the third term $γ ⋅ {M}_{bin} ⋅ {L}_{bin} \left({x}_{bin}, \hat{{x}_{bin}}\right)$ is a loss term designed specifically for binary variables. It evaluates discrepancies between observed binary values x_{bin} and predicted binary outcomes $\hat{{x}_{bin}}$. Again, the mask M_{bin} is used to focus the loss calculation on observed data, with γ serving as a scaling parameter to balance the binary loss.
- the fourth term δ · D_{KL}(q_{ϕ}(z|x) ∥ p(z)) is a regularization component known as the Kullback-Leibler (KL) divergence. This regularization measures the difference between the learned latent distribution q_{ϕ}(z|x) produced by the encoder and a predefined prior distribution p(z). The scaling factor δ controls the strength of regularization in shaping a structured, meaningful latent space representation.

Prior to training the Riemannian Hamiltonian Variational Autoencoder (RHVAE), input data provided in standard file formats such as plain text files (.txt), comma-separated values (.csv), or Excel spreadsheets (.xlsx) may be converted into structured tabular data compatible with neural network training processes.

The preprocessing of the data may begin by reading input data from the provided files and systematically organizing them into structured numerical arrays (tensors). Each column of the resulting structured dataset may represent a specific medical variable, while each row may correspond to an individual patient record or clinical observation. The data variables may be continuous, categorical, or binary, as previously described.

Continuous variables (e.g., patient age, blood pressure, biochemical measurements) may undergo normalization or standardization processes. Normalization techniques can include scaling variables to a standardized range (e.g., min-max scaling between 0 and 1) or standardization (zero mean, unit variance). Such preprocessing facilitates stable and efficient training of the RHVAE by ensuring numerical stability and comparability across different continuous scales.

Categorical variables (e.g., blood types, diagnostic classes, clinical stages) may be preprocessed using appropriate encoding methods such as one-hot encoding or embedding techniques. One-hot encoding transforms categorical variables into binary vectors, allowing the RHVAE to compute categorical probabilities effectively. Alternatively, embedding techniques may map categorical variables into a lower-dimensional continuous representation that better preserves semantic relationships between categories.

Binary variables (e.g., smoker/non-smoker status, disease presence/absence) may typically be encoded as numerical binary values (0 or 1) directly suitable for use with binary cross-entropy loss computations. No further extensive transformations may be required.

After such preprocessing, the structured tabular medical data may be provided as numerical tensors that constitute the inputs for the encoder network of the RHVAE.

The method may further comprise an inference phase in which new synthetic tabular medical data is generated by:
- sampling latent representations from the learned latent space distribution, guided by the Riemannian metric learned during the training phase, and
- decoding said sampled latent representations using the trained decoder, to generate synthetic tabular medical data.

The inference phase corresponds to the step in which new synthetic tabular medical data is generated using the previously trained RHVAE. During this phase, the trained model receives new latent representations sampled from the latent space. These latent representations are subsequently processed by the decoder to generate new synthetic data entries. As a result, the synthetic data produced in the inference phase reflect the statistical and structural characteristics learned from the original medical dataset during the training phase, making them suitable for subsequent clinical analysis, predictive modeling, or medical research applications.

Sampling latent representations refers to the process of selecting new data points from the latent space of the trained RHVAE. The sampling procedure employs the previously learned Riemannian metric to guide this selection process, ensuring latent points are drawn preferentially from regions of high probability density, thus reflecting the statistical and geometric characteristics of the original medical dataset.

The synthetic tabular medical data generated using the above-mentioned method offers significant utility across a spectrum of applications within healthcare, research, and development. These applications leverage the ability of the method to create realistic, statistically coherent data that mirrors the properties of real-world datasets, while overcoming limitations related to data scarcity, accessibility, or sensitivity.

Key areas of application may include:
- Fundamental and Clinical Research: The synthetic data can may serve as a powerful tool for augmenting existing research datasets. In situations involving rare diseases, underrepresented patient subgroups, or limited sample sizes often encountered in clinical studies, the generated data can enlarge the dataset, enabling more robust statistical power for analysis, hypothesis testing (e.g., exploring potential biomarker correlations, analyzing treatment effects in silico, or studying disease progression patterns), and potentially reducing the burden of recruiting extensive control groups in exploratory phases.
- Training and Validating Artificial Intelligence Models: The performance and reliability of Al and machine learning models in healthcare (e.g., for diagnosis, prognosis, risk prediction) are highly dependent on the quality and quantity of training data. The method provides a means to generate large-scale, diverse synthetic tabular datasets that can be used for training, fine-tuning, validating, and benchmarking such models. This is particularly valuable when real data is scarce, imbalanced, or contains sensitive information that restricts its use. Synthetic data can help improve model generalization, reduce overfitting, explore model robustness across varied simulated patient profiles, and serve as a privacy-preserving resource for developing and testing Al algorithms before deployment on real clinical data. It can also be used to deliberately create balanced datasets or datasets emphasizing specific patient subgroups to mitigate biases.

- Development and Testing of Digital Health Technologies: The creation, testing, and regulatory validation of digital medical devices, particularly Software as a Medical Device (SaMD) that relies on patient data inputs, require access to relevant and realistic data. Synthetic tabular data generated by this method can simulate a wide range of patient physiological states, demographic profiles, and clinical scenarios, providing essential input for algorithm development, software testing, performance evaluation, and stress-testing of these technologies. It allows developers to assess how algorithms perform under diverse or rare conditions and can supplement real-world data in dossiers submitted for regulatory approval, demonstrating algorithm robustness and safety across a broader simulated population.

The present document also concerns a computer program product, comprising instructions which, when executed by a processor, cause the processor to implement the above-mentioned method.

The present document also concerns a computer device comprising:
- an input interface to receive tabular medical data,
- a memory for storing at least instructions of a computer program according to the preceding claim,
- a processor accessing the memory for reading the aforesaid instructions and executing then the above-mentioned method,
- an output interface to provide generated tabular medical data.

The input interface refers to hardware and software components of the computer system that receive or acquire medical tabular data for processing. This interface may read data stored locally or remotely, in standard file formats such as plain text files (.txt), comma-separated values (.csv), Excel spreadsheets (.xlsx), or similar structured or semi-structured data files. The input interface may also support real-time data retrieval from medical databases, hospital information systems (HIS), or electronic health record (EHR) systems, ensuring compatibility with commonly used medical data formats and protocols.

The processor refers to the computational component configured to execute instructions stored in memory and carry out the training and inference phases. The processor may include one or several processing units, for instance central processing units (CPUs), graphical processing units (GPUs), or specialized artificial intelligence (AI) processing hardware such as NVIDIA Tensor Cores or Google Tensor Processing Units (TPUs). During operation, the processor executes computations necessary for optimizing neural network weights, updating the Riemannian metric, performing latent-space sampling, and generating synthetic tabular medical data according to the trained RHVAE model, for example.

The output interface refers to the component responsible for delivering the synthetic tabular medical data generated by the trained RHVAE. The output interface may provide the synthetic data in various standardized or tabular formats (e.g., csv, xlsx, txt, json), suitable for further use in clinical research, predictive modeling, or medical analyses. Additionally, the output interface may optionally integrate generated synthetic data directly into existing clinical or research databases, medical dashboards, or visualization tools, ensuring effective interpretation and immediate usability by healthcare practitioners or researchers.

### Brief description of the drawings

Other features, details and advantages will become apparent from the detailed description below, and from an analysis of the attached drawings, in which:
Figure 1 schematically illustrates a computing device according to an embodiment of the present document;
Figure 2 schematically illustrates an embodiment of the computer-implemented method for anonymizing medical data, encompassing both a training phase and an inference phase.

### Detailed description of the drawings

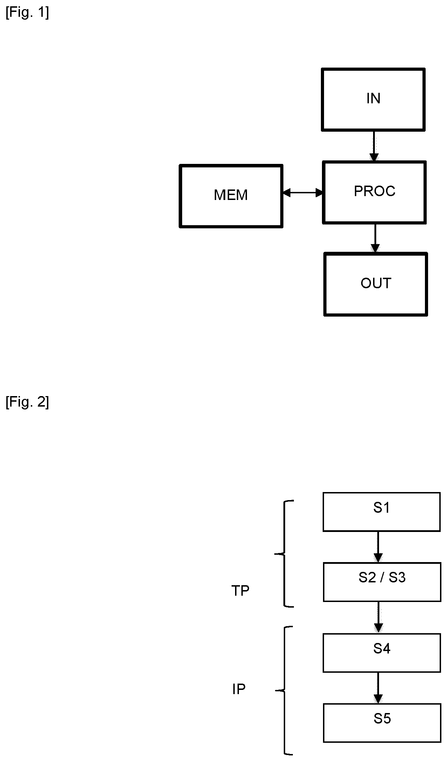

Figure 1 schematically shows an example of a computer device according to the present document.

Said computer device comprises:
- an input interface (IN) to receive tabular medical data,
- a memory (MEM) for storing at least instructions of a computer program according to the present document,
- a processor (PROC) accessing the memory for reading the aforesaid instructions and executing then the method according to the present document,
- an output interface (OUT) to provide generated tabular medical data.

Figure 2 schematically illustrates an embodiment of the computer-implemented method for generating tabular medical data, comprising a training phase (TP) and a subsequent generation or inference phase (IP).

The training phase, depicted generally in Figure 2, involves learning the RHVAE model from a set of original tabular medical data.

This phase comprises providing (S1) the original tabular medical data (potentially preprocessed and including masks for missing values) to the RHVAE framework. During this training phase, the model parameters are optimized (S2). Specifically, using a composite loss function tailored to the heterogeneous data types (continuous, categorical, binary), the weights of the encoder and decoder networks are jointly optimized. This step aims to minimize the reconstruction error according to the defined composite loss, potentially excluding contributions from missing data identified by masks.

Concurrently with the weight optimization, the Riemannian metric defined on the latent space is learned (S3). This metric learning process is guided by the data distribution projected into the latent space and aims to capture its intrinsic geometric structure. The optimization might involve a KL-divergence term to regularize the latent space, with the metric being updated in parallel.

The outputs of this training phase are the trained encoder, the trained decoder, and the learned Riemannian metric, which collectively constitute the trained RHVAE model ready for generating new data.

A subsequent inference phase (IP) involves sampling (S4) latent representations from the learned latent distribution (guided by the learned Riemannian metric, potentially favoring high-density regions) and then decoding these latent representations using the trained decoder to generate (S5) new synthetic tabular medical data, which is the output of the generation process.

## Claims

1. A computer-implemented method for generating tabular medical data using a Riemannian Hamiltonian Variational Auto Encoder (RHVAE), said method comprising a training phase (TP),
said RHVAE comprising said RHVAE comprising an encoder configured to project said tabular medical data into a latent space, and a decoder configured to reconstruct tabular medical data from latent representations,
said tabular data comprising tabular variables, continuous variables and/or categorical variables,
the training phase comprising:
- defining a composite loss function adapted to the different types of tabular variables, said composite loss comprising a first loss function, for example an L1 loss, for continuous variables, a second loss function, for example a cross-entropy loss, for categorical variables, a third loss function, for example a binary cross-entropy loss for binary variables,
- training said RHVAE by jointly
-- optimizing (S2) weights of said encoder and decoder using said composite loss function, and
-- learning (S3) a Riemannian metric on the latent space so as to capture the intrinsic geometry of said tabular medical data.

2. The method according to the preceding claim, wherein said Riemannian metric guides a Hamiltonian Monte Carlo (HMC) sampling procedure in the latent space, so that the sampling path favors high-density regions corresponding to the distribution of the tabular medical data.

3. The method according to any of the preceding claims, wherein missing data in the tabular medical data are identified via a mask and excluded from the reconstruction error calculation in said composite loss function.

4. The method of any of the preceding claims, wherein optimizing the encoder and decoder comprises applying a KL-divergence regularization term between a learned latent distribution and a predefined prior, the Riemannian metric being updated in parallel to reflect the curvature of the latent space based on said tabular medical data.

5. The method according to any of the preceding claims, further comprising an inference phase in which new synthetic tabular medical data is generated by:
- sampling latent representations from the learned latent space distribution, guided by the Riemannian metric learned during the training phase, and
- decoding said sampled latent representations using the trained decoder, to generate synthetic tabular medical data.

6. A computer program product, comprising instructions which, when executed by a processor, cause the processor to implement the method according to any of the preceding claims.

7. A computer device comprising:
- an input interface (IN) to receive tabular medical data,
- a memory (MEM) for storing at least instructions of a computer program according to the preceding claim,
- a processor (PROC) accessing the memory for reading the aforesaid instructions and executing then the method according to any of the claims 1 to 5,
- an output interface (OUT) to provide generated tabular medical data.
